Europäisches Patentamt

European Patent Office

Office européen des brevets

Publication number: **0 274 868**
**A1**

# EUROPEAN PATENT APPLICATION

Application number: **87310836.9**

Date of filing: **09.12.87**

Int. Cl.4 **G01F 1/00 , B63C 11/24 , A61M 16/00**

Priority: **13.12.86 GB 8629825**
**02.04.87 GB 8707869**

Date of publication of application:
**20.07.88 Bulletin 88/29**

Designated Contracting States:
**BE DE FR GB IT NL SE**

Applicant: **Clausen, Kaldager Instruments Limited**
**Unit 2 Robert Leonard Industrial Centre**
**Howe Moss Drive**
**Dyce Aberdeen AB2 0GL Scotland(GB)**

Inventor: **Russell, Ian Joseph**
**The Gables Hatton of Fintray**
**Aberdeenshire Scotland(GB)**

Representative: **Pattullo, Norman et al**
**Ian G. Murgitroyd and Company Mitchell**
**House 333 Bath Street**
**Glasgow G2 4ER Scotland(GB)**

## Measuring apparatus.

A gas flow measuring system comprising a gas flow conduit (32; 132) for carrying a flowing gas mixture of known constituents in unknown proportions, characterised by a gas analyser (34; 134) coupled to the conduit (32; 132) for measuring the specific gravity of the flowing gas mixture, a mass flowmeter (38; 138) coupled to the conduit (32; 132) for measuring the mass flow rate of the flowing gas mixture, and a measurement correlator (58; 158) for correlating the measurements of specific gravity and of mass flow rate to produce a resultant signal (60; 160) representative of the volumetric flow rate of the flowing gas mixture.

FIG. 1

EP 0 274 868 A1

## Measuring Apparatus

This invention relates to gas flow measuring systems, particularly systems for measuring volumetric gas flow rates and preferably also total volumetric gas flow.

There are circumstances where it is desirable or essential to know instantaneous or short-term volumetric gas flow rates, and preferably also the total volume of gas transferred in the longer term or over a predetermined period of time. For example, the monitoring of breathing gas mixture consumption rates in life support systems can give indications of metabolic rates and system leaks, while a measurement of total volumetric gas flow can be employed for determining overall efficiency and total cost of consumed gases.

Inspection, maintenance, and repair operations in the offshore oil industry may require divers to operate for extended periods of time in water of great depth. For well known reasons, divers working at great depth and correspondingly great pressure cannot breathe ordinary air. Consequently, such divers must be supplied with a mixture of helium and oxygen, the correct percentage of oxygen being a function of the required pressure in the diver's helmet.

Helium and oxygen of the necessary purity and in the substantial amounts needed for diving operations (including preceding preparation of the diver and succeeding depressurisation) are very expensive. Helium consumption is minimised by supplying the diver through a closed-loop breathing system in which the exhaled gases are passed through a regenerator which removes carbon dioxide (and preferably also other undesirable gases and vapours) and replaces oxygen depleted by breathing, while leaving the helium content theoretically intact.

Such closed loop breathing systems are known, but the retention of helium is almost inevitably less than perfect. Loss of helium also occurs through leakage, which will be exacerbated by the hyperbaric operating pressures.

As mentioned already, the correct proportion of oxygen in the helium oxygen mixture is not fixed, but is a function of operating conditions. Further complications in maintenance of the correct proportions of helium and oxygen arise from the injection of make-up gases into the system to compensate for leaks and for any necessary increase in system pressure.

It is therefore highly desirable, for reasons including health and safety, to know the instantaneous proportions and volumetric flow rates of the gas mixture as supplied to the diver and returned from the diver, the input and output of the regenerator, and the system make-up gases, both during full-depth work by the diver and when the diver is in the diving bell or other saturation chamber system where the diver is purged of nitrogen prior to diving and controllably depressurised after diving. It is also desirable, for reasons including efficiency monitoring and cost control, to know the total volumetric flows that have occured over the entire diving operation (or any distinct stage of the diving operation) since these flow totals can give indications of the efficiency of various parts of the system and the finanical costs of the gases. It is also clearly desirable to have an accurate measure of the total volume of bulk gases delivered by a gas manufacturer supplier to the gas user (in a manner analogous to the metering of bulk supplies of liquid fuels). Such measurements are not at present readily obtainable with acceptable accuracy due to the limitations of known forms of gas flow measuring equipment.

It is an object of the invention to provide a gas flow measuring system for measuring volumetric gas flow rates, and which may be adapted to measure total volumetric gas flow.

According to the present invention there is provided a gas flow measuring system comprising a gas flow conduit for carrying a flowing gas mixture of known constituents in unknown proportions, a gas analyser coupled to the conduit for measuring the specific gravity of the flowing gas mixture, a mass flowmeter coupled to the conduit for measuring the mass flow rate of the flowing gas mixture, and a measurement correlator for correlating the measurements of specific gravity and of mass flow rate to produce a resultant signal representative of the volumetric flow rate of the flowing gas mixture.

The flowing gas mixture is preferably a binary gas mixture, for example a mixture of helium and oxygen free of other gases in quantities sufficient to cause substantial errors in the measurements.

Other binary gas mixtures may consist of oxygen-enriched air, i.e. nitrogen and oxygen with the oxygen in excess of 21 volume percent (for therapeutic purposes), and anaesthetic analgesic gas mixtures such as nitrous oxide and oxygen. Alternatively, the flowing gas mixture may contain more than two components, provided that the components can be allocated to two groups such that the components within each group remain in mutually fixed proportions and only the proportion of one group in toto varies with respect to the other group in toto as respective fractions of the overall mixture. In addition to breathing gas mixtures, the flowing gas mixture may comprise one or more components intended for industrial processes such as chemical engineering.

The gas analyser is preferably an electrochemical gas analyser as described in either of United States Patents 3.429.796 and 3.767.552. Such analysers are particularly suited to the measurement of oxygen content of gas mixtures, and can readily determine the proportion of oxygen in a helium.oxygen mixture to produce a representative electrical output signal. Since the specific gravities of pure helium and of pure oxygen are each known (at standardised pressure and temperature), measurement of the oxygen content of a helium oxygen mixture can be directly converted to a specific gravity reading. Such a gas analyser is preferably calibrated against a gas "mixture" which is in fact pure helium, but any suitable gas mixture of known proportions can be employed for calibration. (If the non-oxygen component(s) of the gas mixture is not helium, then the appropriate gas would be used for calibration, such as nitrogen for subsequent analysis of oxygen-enriched air).

The mass flowmeter is preferably a Coriolis force mass flowmeter as described in United Kingdom Patent 2.001.759, which is capable of directly measuring mass flow rate through a conduit to produce a representative electrical ouput signal. Another Coriolis-type mass flowmeter is described in United States Patent 4.691.578, and this flowmeter also produces an electrical output signal which is representative of the mass flow rate of fluid flowing through a conduit.

The measurement correlator generally operates by dividing the mass flow rate measurement by the specific gravity measurement to give the required volumetric flow rate measurement. In the case where the specific gravity measurement is made directly or indirectly (e.g. from oxygen content measurement) at the operating pressure of the system, the specific gravity measurement will pertain to the gas pressure within the gas flow conduit, which may be substantially above atmospheric pressure when the flowing gas mixture is a breathing mixture for a diver. Consequently, the volmetric flow rate measurement will be an actual volumetric flow rate pertaining to the actual pressure and temperature at the location where the specific gravity is measured. This actual volumetric flow rate measurement is preferably also presented as an equivalent volumetric flow rate at a standardised pressure and temperature, suitably 1 bar or nominal atmospheric pressure at sea level, and zero degrees Celsius. Conversion of the volumetric flow rate measurement is achieved by measuring the ratios of actual gas pressure and temperature in the gas flow conduit to the standardised pressure and temperature, and multiplying the actual flow rate measurement by these ratios or by other suitably related factors. Measurement of actual gas pressure and temperature can be performed by any suitable devices or equipment, which may be an integral part of the gas analyser or separate therefrom.

A potentially simpler procedure for obtaining the equivalent volumetric flow rate at standardised conditions is to make the specific gravity measurement under these standardised conditions. Thus in the case where the gas analyser functions by analysing oxygen content, gas samples can be obtained by bleeding a small but representative flow of gas mixture from the gas conduit through a pressure regulator or other pressure reducing device, and presenting the sampled gases to the gas analyser substantially at the standardised pressure, and at a temperature which does not differ from the standardised temperature by an amount which will produce significant errors in the resultant analytical measurements.

Measurement of volumetric flow rate can be converted to a measurement of total volumetic flow in given period of time by integration of the instantanenous volumetric flow rate measurements throughout that period of time. Integration of the appropriate electrical signals can be carried out by any suitable electronic circuit, either analogue or digital. Indications of total volumetric flow may be given both for actual volume, and for equivalent volume at the standardised pressure and temperature.

In the case where the gas flow measuring system is applied to measuring the volumetric flow rate of a breathing gas mixture supplied to a diver in a closed-loop breathing system into which a make-up gas mixture may be injected, the gas flow measuring system is preferably duplicated and the second gas flow measuring system is coupled in series with the make-up gas mixture supply such that the volumetric flow rate and composition of the injected make-up gas mixture can be measured in like manner to the gas mixture flowing around the closed loop.

The make-up gas mixture can be injected into the closed loop upstream or downstream of the system applied to measuring the gas flow around the closed loop.

Embodiments of the present invention will now be described by way of example, with reference to the accompanying drawings wherein:-

Fig. 1 is a schematic circuit diagram of a first embodiment of gas flow measuring system in accordance with the invention;

Fig. 2 is a schematic circuit diagram of a second embodiment of gas flow measuring system in accordance with the invention, in the form of a modification of the first embodiment; and

Fig. 3 is a schematic circuit diagram of a modified form of gas analyser suitable for substitution into the first and second embodiments of the invention.

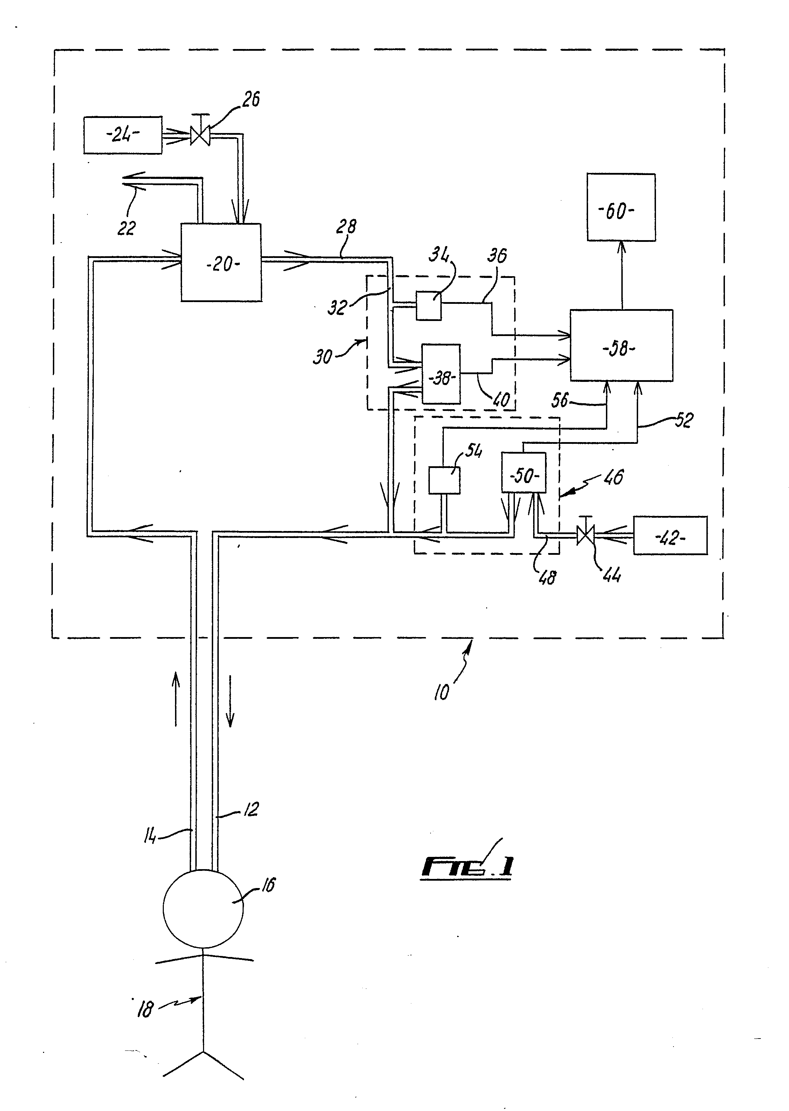

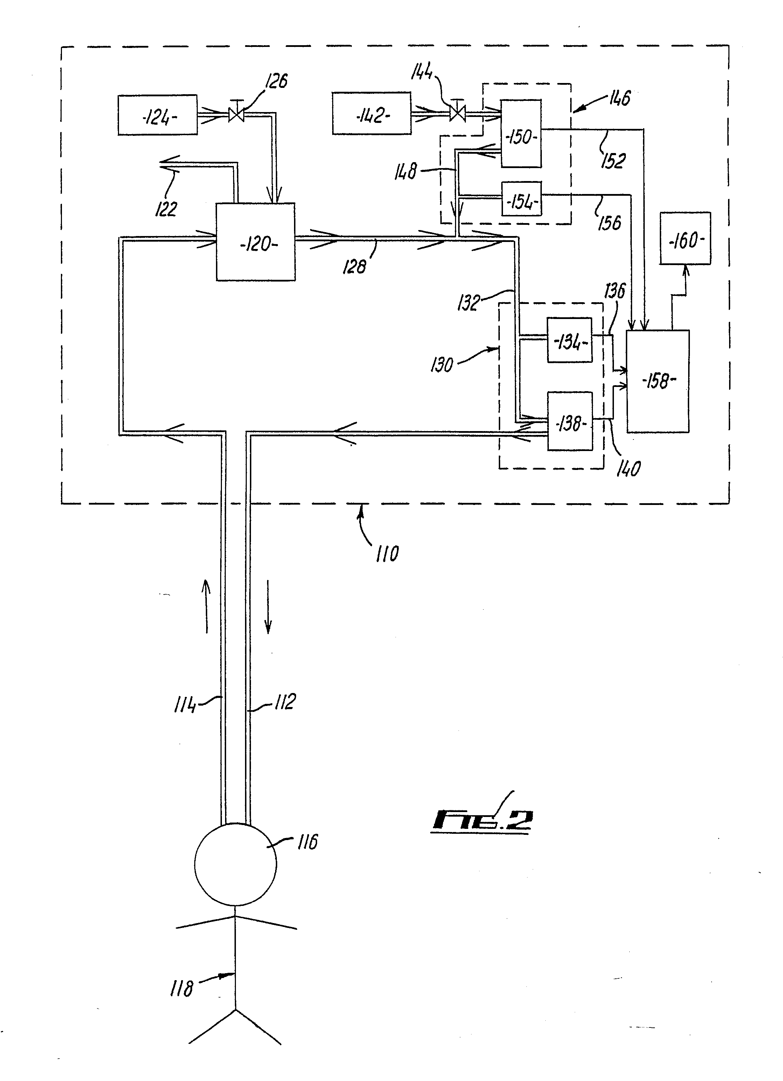

Figs. 1 and 2 are both highly schematic diagrams showing functional blocks interconnected by gas conduits and electrical connections.

In Fig. 1, a diver's breathing gas mixture supply system 10 is mounted on a ship or oil rig (not shown) and is coupled through a supply hose 12 and a return hose 14 to the helmet 16 of a diver 18. The breathing gas mixture is helium and oxygen, with 2-21 volume percent of oxygen according to requirements. In normal operation, the system 10 is a closed loop breathing system in which exhaled breathing gases are returned from the helmet 16 via the return hose 14 to a regenerator 20. Within the regenerator 20, carbon dioxide is removed from the exhaled gases by any suitable method, and either vented through a waste gas exhaust 22 or internally absorbed in a cannister of soda lime (not shown). Since the oxygen content of the breathing mixture is depleted by breathing, the proportion of oxygen is returned to the correct level by injection from an oxygen supply 24 through a control valve 26. The regenerator 20 preferably also regulates the humidity of the regenerated breathing gas mixture delivered at its output conduit 28; temperature may additionally be regulated.

Before the output of the regenerator 20 is fed back into the diver's supply hose 12, the regenerated gas mixture has its volumetric flow rate measured in a first gas flow measuring system 30 which has a gas flow conduit 32 coupled to the regenerator output conduit 28. A gas analyser 34 is coupled to the gas flow conduit 32, to measure the oxygen content of the helium/oxygen mixture and to produce a representative electrical output signal on an output signal lead 36. The gas analyser 34 is an electrochemical oxygen analyser as described in either of United States Patents Nos. 3,429,796 and 3,767,552.

The gas flow conduit 32 within the first measuring system 30 is next coupled to a mass flowmeter 38 which is a Coriolis-type mass flowmeter in which all the gas flows through a curved tube driven into vibratory motion. Full details of the preferred form of the mass flowmeter 38 are given in United Kingdom Patent No. 2,001,759, and it may be constituted by the commerically available "Micomotion D12 meter". In operation, the mass flowmeter 38 produces a mass-flow-representative electrical output signal on an output signal lead 40.

The continuation of the gas flow conduit 32 beyond the mass flowmeter 38 conveys the flow-measured gas mixture out of the first flow measuring system 30 and into the surface end of the diver's supply hose 12.

Although the breathing system described above is nominally a closed loop system, there will in practice be a need to add gases to the system, either intermittently or continuously. This need arises from a number of causes, including leaks, deliberate venting or blowdown, and general increases in system pressure (for example, due to descent of the diver 18). The make-up gases are supplied as required from a pressurised gas source 42 containing a helium oxygen mixture of known proportions. Make-up gas supply from the gas source 42 is regulated by a control valve 44. For example, if the diver 18 is operating at a depth of 100 metres at which the ambient water pressure is about 10 bar, the pressure required at the inlet end of the supply hose 12 is about 18 bar, and the pressure of make-up gases in the gas flow conduit 48 will be maintained at the same 18 bar pressure to match the pressure of regenerated breathing gas mixture in the gas flow conduit 32.

The volumetric flow rate of the make-up gases from the gas source 42 is measured by passing the make-up gases through a second gas flow measuring system 46. The flow measuring system 46 is similar to, and may be identical to, the first gas flow measuring system 30.

The supply of make-up gases released through the control valve 44 enters a gas flow conduit 48 passing completely through the flow measuring system 46. The gas flow conduit 48 first carries the make-up gases through a Coriolis-type mass flowmeter 50 which is similar or identical to the mass flowmeter 38. In operation, the mass flowmeter 50 produces a mass-flow-representative electrical output signal on an output signal lead 52. The gas flow conduit 48 conveys the make-up gases from the mass flowmeter to a gas analyser 54 which measures the oxygen content of the make-up gases to produce a representative electrical output signal on an output signal lead 56. The make-up gases then leave the second flow measuring system 46 and enter the surface end of the supply hose 12 at a point immediately downstream of the first flow measuring system 30 (which is delivering the regenerated breathing gas mixture to the inlet or surface end of the supply hose 12).

The output signal leads 36, 40, 52, and 56 are each connected to a measurement correlator 58. Prior to operational use, the supply system 10 will have been calibrated by supplying pure helium (or a helium oxygen mixture of known proportions) at a suitable known pressure and temperature to the gas analysers 34 and 54, to cause calibration measurements to be produced and fed to the correlator 58 along the leads 36 and 56. The correlator 50 is thereby enabled during operational use to convert the oxygen content measurement signals on the leads 36 and 56 to equivalent specific gravity measurement signals for the gas mixtures in the gas flow conduits 32 and 48 respectively.

The correlator 58 also receives direct mass flow rate measurements via the leads 40 and 52 from the mass flowmeters 38 and 50 respectively.

From these indirect specific gravity measurements and the direct mass flow measurements, the correlator 58 can correlate the measurements to produce respective actual volumetric flow rates in the gas flow conduits 32 and 48, and by further correlation, the volumetric flow rate of the gases entering the supply hose 12. These various volumetric flow rates can be individually or collectively indicated on a suitable display device 60 adjacent to the measurement correlator 58 and/or at a separate diving control station (not shown).

Pressure and temperature measurements may be taken by suitable transducers (not illustrated) either incorporated in the gas analysers 34 and 54 or otherwise respectively coupled to the gas flow conduits 32 and 48, to feed respective pressure and temperature measurement signals to the correlator 58. Such pressure and temperature measurements can enable the correlator 58 to convert the actual volumetric flow rates to equivalent volumetric flow rates at standardised pressure and temperature (e.g. 1 bar and zero degrees Celsius). These equivalent volumetric flow rates can be displayed in addition to or as an alternative to display of the measurement of actual volumetric flow rates.

The measurement correlator 58 preferably performs time integration of the volumetric flow rate measurements (whether actual, standardised, or both) to give total volumetric flow over the period of integration, respectively expressed as actual volume, standardised volume, or both.

The correlator 58 preferably correlates the various measurements of volumetric flow rates and volumetric flows to give indications of the respective performances of the various parts of the system 10 and of the diver 18, plus indications of possible or actual leaks from any part of the system 10, the hoses 12 and 14, and the diver's helment 16. (Performance of the diver 18 can be monitored by measurement of his oxygen consumption, which is closely related to his rate of physical exertion, while an abnormally high or low oxygen consumption may indicate a medical emergency). An abnormally high volumetric flow rate of make-up gases 42 usually indicates an unacceptably serious leak.

Fig. 2 illustrates a diver's breathing gas mixture supply system which is a modification of the system shown in Fig. 1. Those parts of the system of Fig. 2 which individually correspond to the system of Fig. 1 are given the same reference numeral as in Fig. 1, but prefixed by a "1" (i.e. the same reference numeral as in Fig. 1, plus "100"). Since so much of the system of Fig. 2 is structurally and functionally indentical to the system of Fig. 1, the following description of Fig. 2 is largely confined to the differences of the system of Fig. 2 relative to the system of Fig. 1; for a detailed description of the system of Fig. 2, reference should be made to the corresponding parts of Fig. 1.

The principal difference between the system of Fig. 2 and the system of Fig. 1 is that in Fig. 2, the make-up gases from the make-up gas source 142 enter the gas conduit leading to the inlet end of the diver's supply hose 112 upstream of the gas flow conduit 132 within the first gas flow measuring system 130. As in Fig. 1, the volumetric flow rate of the make-up gases are measured by means of the second gas flow measuring system 146. However, the first gas flow measuring system 130 now measures the total volumetric gas flow rate of the gas mixture entering the surface end of the diver's supply hose 112. (In the Fig. 1 system, the first gas flow measuring system 30 measured only the volumetric gas flow rate of the regenerated breathing gas mixture leaving the regenerator 20, and the volumetric gas flow rate of the make-up gases was independently measured by the second gas flow measuring system 46). Consequently, the measurement correlator 158 is suitably modified to take account of the qualitatively different volumetric gas flow rate measurements produced by the first gas flow measuring system 130, which now represent the total supply to the diver 118 (neglecting leakage).

As further possible modifications of the invention, any one or all of the gas analysers 34, 54, 134, and 154 may be replaced by devices that directly measure the specific gravity of the gas mixture flowing in the respective gas conduit to which they are coupled, the measurement correlator 58 and/or 158 being appropriately modified. As an alternative to use of pure helium for calibration of the gas analysers or other devices for directly or indirectly measuring specific gravity, calibration can be carried with any suitable gas mixture of known composition. Mass flowmeters operating on a principle other than measurement of Coriolis forces can be employed in place of any one or all of the mass flowmeters 38, 50, 138, and 150.

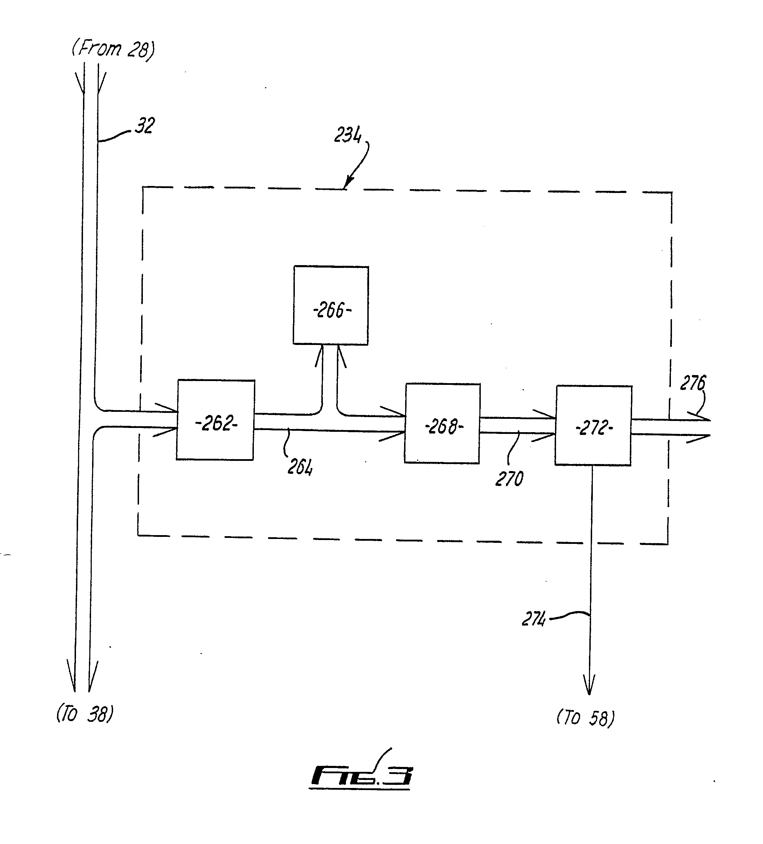

As a still further modification of the present invention, any one or all of the gas analysers 34, 54, 134, and 154 may be replaced by the system shown in Fig. 3. In the first and second embodiments of Figs 1 and 2, the above referenced gas analysers operated at the hyperbaric internal pressure of the supply system 10 or 110 respectively, and their respective output readings required transformation to give equivalent readings at the standardised pressure of 1 bar. The system of Fig. 3 is an arrangement for obtaining an oxygen content measurement at approximately atmospheric pressure, and hence for obtaining an indirect reading of specific gravity at atmospheric pressure without the previously mentioned transformations of measurement in accordance with actual pressure and temperature (and the additional complexity of measuring such parameters).

Referring now to Fig. 3 in detail, this schematically illustrates a gas analyser circuit 234 intended to be a modification or replacement of the gas analyser circuit 34 of Fig. 1. The gas analyser circuit 234 is coupled to the gas flow conduit 32 in like manner to the gas analyser 34 of Fig. 1. Within the circuit 234, gas from the conduit 32 is first reduced in pressure within a pressure regulator 262, which may be constitued by the commericaly available "Tescom regulator". The pressure-regulated output from the regulator 262 is delivered along a conduit 264 to a pressure indicator 266 and to a variable-area flowmeter 268. The pressure indicator 266 and the variable-area flowmeter 268 are utilised to set the pressure regulator 262 to deliver an output pressure in the conduit 264 which is just above ambient atmospheric pressure around the supply system 10 when the volume passing through the flowmeter 268 is in the range 1-1.5 litres per minute (which is a small aliquot of the gas mixture flowing through the conduit 32).

The gas passing through the flowmeter 268 is delivered along an output conduit 270 to an oxygen analyser 272 which is preferably in the same form as the analyser 34 (i.e. as in either of US Patents Nos. 3,429,796 and 3,767,552), subject to any necessary or desirable modifications for working at ambient atmospheric pressure in contrast to the previously described hyperbaric pressure. The oxygen-content-representative electrical output signal from the analyser 272 is delivered on an output signal lead 274 which is connected in place of the lead 36 shown in Fig. 1. Gases which have passed through the analyser 272 are exhausted as waste to ambient atmosphere through a vent 276.

The correlator 58 will be modified as necessary to deal with the signal from the substitute gas analyser circuit 234 on the basis that it now represents the oxygen content of a representative sample of the gas mixture flowing along the conduit 32, but already transformed to an equivalent measurement at atmospheric pressure (assumed equal to the standardised pressure). Thus the readings on the display device 60 will now be the measurement of standardised volumetric flow rate and standardised total volumetric flow.

The gas analyser 54 is preferably modified or substituted in like manner to the modification or substitution of the gas analyser circuit 234 for the gas analyser 34. Similar modifications can be made to either or both the gas analysers 134 and 154 in the second embodiment shown in Fig. 2.

The systems of Figs 1 and 2 have been described with respect to monitoring the supply and consumption of a diver's breathing gas mixture during diving operations. The gas flow measuring system of the invention is not restricted to such application, and a number of other uses are possible; for example, (1) monitoring the reclamation and purification of spent breathing gases after a diving operation is concluded, such as when the gases are being purified by a process not employed during diving operations, e.g. to remove metabolites other than carbon dioxide; (2) accurate volumetric metering of the delivery of bulk supplies of gases from a gas manufacturer supplier; (3) monitoring the volumetric flow rates of oxygen-enriched air employed for medical or therapeutic purposes, as in hyperbaric oxygen therapy or to aid the breathing of hospital patients at atmospheric pressure; (4) monitoring the volumetric flow rates of anaesthetic analgesic gas mixtures, whether in closed-loop breathing systems or in open-loop breathing systems; and (5) monitoring the volumetric flow rates of gas mixtures employed for industrial purposes, e.g. in chemical engineering.

The invention can therefore provide a device which will measure flowrates of gases in volumetric terms, express the readings in standard form e.g. standard cubic feet per minute, and totalise the flow e.g. standard cubic feet, wherein "standard" is the volume that the gas would occupy under standard conditions of temperature and pressure.

Using conventional Physics we know:

$$\frac{\text{Mass}}{\text{density}} = \text{Volume}$$

Adapting this formula:

$$\frac{\text{Mass Flow Rate}}{\text{density } (\rho)} = \text{Volumetric Flow Rate}$$

Remembering that mass is unaffected by pressure or temperature we can make a further modification:

$$\frac{\text{Mass Flow (kg per minute)}}{\rho \text{ (standard)}} = \text{Volume Flow (metres}^3 \text{ per minute) (standard)}$$

where mass flow is derived using a coriolis mass flow meter and $\rho$ (standard) is derived using a gas analysis technique.

$\rho_S = \rho_{A(S)} + \rho_{B(S)} + \rho_{C(S)}$ etc

As an example, if we have a mixture of oxygen and helium where $O_2 = 10\%$ and $He = 90\%$, then:

$\rho_{S_{HE02}} = 10\%(\rho O_2) + 90\%(\rho_{He})$

From this we can say that to determine the density of the mixture of a gas with 2 constituents we need only know their relative proportions i.e. measure one and derive the density of the whole by calculation.

Other modifications and variations can be made within the scope of the invention as defined in the appended claims.

## Claims

1. A gas flow measuring system comprising a gas flow conduit (32; 132) for carrying a flowing gas mixture of known constituents in unknown proportions, characterised by a gas analyser (34; 134) coupled to the conduit (32; 132) for measuring the specific gravity of the flowing gas mixture, a mass flowmeter (38; 138) coupled to the conduit (32; 132) for measuring the mass flow rate of the flowing gas mixture, and a measurement correlator (58; 158) for correlating the measurements of specific gravity and of mass flow rate to produce a resultant signal (60; 160) representative of the volumetric flow rate of the flowing gas mixture.

2. A gas flow measuring system as claimed in Claim 1 and wherein the flowing gas mixture is a binary gas mixture, characterised in that the gas analyser (34; 134) measures the proportion of the binary gas mixture that is constituted by one of the two components of the binary gas mixture.

3. A gas flow measuring system as claimed in Claim 2 and wherein said one of the two components of the binary gas mixture is oxygen, characterised in that the gas analyser (34; 134) is an electrochemical oxygen analyser producing an electrical output signal directly from electrochemical reaction of components of the analyser with the oxygen content of the gas mixture being analysed in said analyser.

4. A gas flow measuring system as claimed in any preceding claim characterised in that the mass flowmeter (38; 138) is Coriolis-type mass flowmeter wherein the flowing gas is carried through a curved conduit which is cantilever-mounted and oscillated at right angles to the plane of curvature such that Coriolis forces induced by gas flowing in the conduit induce phase differences in the vibratory motions of different parts of the conduit, said phase differences being dependent on the mass flow rate of gas in said curved conduit whereby measurement of said phase differences gives a measurement of mass flow rate.

5. A gas flow measuring system as claimed in any preceding claim, characterised in that the measurement correlator (58; 158) operates by dividing the mass flow rate measurement by the specific gravity measurement to give the required volumetric flow rate measurement.

6. A gas flow measuring system as claimed in any preceding Claim characterised by a pressure regulator (262) coupled between the gas flow conduit (32) and the gas analyser (272) to reduce the pressure of the gas mixture being analysed to a standardised pressure such that the measurement of specific gravity is a measurement at the standardised pressure and the measure of volumetric flow rate (60) is a measure of the volumetric flow rate at the standardised pressure.

7. A gas flow measuring system as claimed in any preceding Claim characterised in that the measurement correlator (58; 158) is adapted to perform a time integration of instantaneous volumetric flow rate to produce a measurement of total volumetric flow over a period of time.

8. A gas flow measuring system as claimed in any preceding Claim wherein the flowing gas mixture is a breathing gas mixture circulating in a closed-loop breathing system, and additional quantities of breathing gas mixture are intermittently or continuously injected into the closed-loop breathing system as make-up gases, characterised in that the gas flow measuring system is duplicated (30, 46; 130, 146) for separate volumetric flow measurement of gas mixture flowing around the closed-loop breathing systen and of gas mixture injected into the closed-loop breathing system as make-up gases.

9. As gas flow measuring system as claimed in Claim 8, wherein the closed-loop breathing system includes a breathing gas mixture regenerator (20), and the gas flow measuring system (30) which measures the volumetric flow rate of gases circulating in the closed loop is connected between the regenerator (20) and the breathing gas consumer (18), characterised in that the make-up gases (42) are injected through the other gas flow measuring system (46) into the closed loop at a point between the loop flow measuring system (30) and the consumer (18).

10. A gas flow measuring system as claimed in Claim 8, wherein the closed-loop breathing system includes a breathing gas mixture regenerator (120), and the gas flow measuring system (130) which measures the volumetric flow rate of gases circulating in the closed loop is connected between the regenerator (120) and the breathing gas consumer (118), characterised in that the make-up gases (142) are injected through the other gas flow measuring system (146) into the closed loop at a point between the regenerator (120) and the loop flow measuring system (130).

Fig.1

**FIG.2**

(From 28)

32

234

-266-

-262-

264

-268-

270

-272-

276

274

(To 38)

(To 58)

FIG.3

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | PATENT ABSTRACTS OF JAPAN, vol. 9, no. 292 (P-406)[2015], 19th November 1985; & JP-A-60 129 642 (MITSUBISHI JUKOGYO K.K.) 10-07-1985 * Abstract * | 1,2 | G 01 F   1/00 B 63 C   11/25 A 61 M   16/00 |
| A | Idem | 5,6 | |
| Y | US-A-3 593 735  (REIHER) * Figures; abstract; column 1, line 71 - column 2, line 37; column 4, line 52 - column 5, line 12 * | 1,2 | |
| .Y A | | 3 8-10 | |
| Y | US-A-3 767 552  (LAUER) * Figures; abstract * | 3 | |
| A | US-A-4 187 721  (SMITH) * Figures; column 7, lines 19-38; column 9, lines 10-55 * | 4,5,7 | TECHNICAL FIELDS SEARCHED (Int. Cl.4) |
| · A | US-A-4 233 842  (RAEMER et al.) * Figures; abstract; column 1, lines 39-50 * | 1,2,8 | G 01 F B 63 C A 61 M B 62 B |

The present search report has been drawn up for all claims

| Place of search | Date of completion of the search | Examiner |
|---|---|---|
| THE HAGUE | 14-03-1988 | NUIJTEN E.M. |

EPO FORM 1503 03.82 (P0401)